# EUROPÄISCHE PATENTSCHRIFT

(11) **EP 2 746 771 B1**
(45) Veröffentlichungstag und Bekanntmachung des Hinweises auf die Patenterteilung: **16.05.2018**
(21) Anmeldenummer: 13198665.5
(22) Anmeldetag: 19.12.2013
(51) Int. Cl.: G01N 33/53, G01N 21/00, A61M 1/14

(54) **VORRICHTUNG ZUR BESTIMMUNG VON ABFALLPRODUKTEN WIE INDOXYL SULFATE IN DER DIALYSE**
DEVICE FOR DETERMINING WASTE PRODUCTS SUCH AS INDOXYL SULPHATES IN DIALYSIS
DISPOSITIF DE DÉTERMINATION DE DÉCHETS TELS QUE L'INDOXYL SULFATE DANS LA DIALYSE

(30) Priorität: 20.12.2012 DE 102012112790
(43) Veröffentlichungstag der Anmeldung: 25.06.2014
(73) Patentinhaber: B. Braun Avitum AG, 34212 Melsungen (DE)
(72) Erfinder: Meibaum, Jörn, 34225 Baunatal (DE); Strohhöfer, Christof, Dr., 34123 Kassel (DE)
(74) Vertreter: Winter, Brandl, Fürniss, Hübner, Röss, Kaiser, Polte - Partnerschaft mbB

(56) Entgegenhaltungen:
- EP-A1- 2 397 167
- EP-A1- 2 510 958
- WO-A1-2012/000521
- WO-A1-2012/022304

## Beschreibung

Die Erfindung bezieht sich auf eine Vorrichtung zur Bestimmung von Abfallprodukten während einer Dialysebehandlung.

Bei Patienten mit eingeschränkter oder fehlender Nierenfunktion werden Abfallprodukte des natürlichen Stoffwechsels einschließlich urämischer Toxine durch Dialyseverfahren entfernt, wobei die Entfernung der Stoffe aus dem extrakorporal geführten Blut durch den Kontakt des Blutes mit einer Dialysierflüssigkeit erfolgt. Die Dialysierflüssigkeit ist mit verschiedenen Salzen versetzt und ruft somit diffusive und konvektive Effekte hervor, welche für den Stofftransport vom Blut über eine Membran in die Dialysierflüssigkeit verantwortlich sind. Das gereinigte Blut wird anschließend dem Patienten wieder zugeführt.

Das Maß für die Dialyse eines Patienten kann auf der Basis der subjektiven Einschätzung der Gesundheit des Patienten erfolgen, was allerdings recht ungenau ist. Um einen ausreichenden Dialyseeffekt zu gewährleisten, sollte der Dialyseerfolg präziser quantifiziert werden. Hierdurch lässt sich dann auch ein zu hohes, kostenintensives und den Patienten evtl. belastendes Maß an Dialyse vermeiden. Um die Dialysetherapie effizienter zu gestalten, ist es wünschenswert, die Dialyseeffizienz in Echtzeit kontrollieren zu können und so die Behandlung durch Anpassung der Parameter der Dialysemaschine manuell oder automatisch steuern zu können.

Zur Gewährleistung einer adäquaten Dialysetherapie wurde ein *Kt*/*Vᵤᵣₑₐ*-Modell entwickelt. Hierbei wird Harnstoff (Urea) ausgewertet, der eines der Abfallprodukte darstellt, die zur Bestimmung einer adäquaten Dialysetherapie herangezogen werden können. Hierbei bezeichnet *K* die Reinigungsleistung des Dialysators von *Urea* aus dem Blut in *ml*/*min, t* die Behandlungszeit in *min* und *V* das Verteilungsvolumen von *Urea* in *ml* im menschlichen Körper, welches direkt in Zusammenhang mit dem Patientengewicht steht. Der dimensionslose Faktor *Kt*/*Vᵤᵣₑₐ* definiert die Reduktion von Harnstoff-Stickstoff im Blut bei einer dreimaligen Behandlung pro Woche.

Neben der Reduktion von Harnstoff ist jedoch auch das Entfernungsverhalten von anderen Stoffen wie proteingebundenen Substanzen wie P-Kresol, P-Kresyl Sulfat, oder Indoxyl Sulfat oder mittelgroßen Molekülen im Bereich von 500 bis ca. 50000 Da zur Bewertung der Dialyseeffizienz wichtig, da diese maßgeblich für die Pathologie bei einer Urämie sind. Zu mittelgroßen Molekülen zählen u.a. kleine Proteine und Peptide wie β₂-Mikroglobulin, Cystatin C, Retinol-Binding Protein RBP oder beispielsweise Complement Faktor D (VFD).

Absorbanz- und Lumineszenzmessungen sind gängige analytische Verfahren zur Untersuchung von Molekülen im elektromagnetischen Spektralbereich. Lumineszenzmessungen werden üblicherweise zum Nachweis von Proteinen, welche über anregungsfähige Strukturen wie z.B. Fluorophore verfügen, eingesetzt. Neben Proteinen verfügen auch einige Peptide und andere chemische Substanzen über die Fähigkeit, elektromagnetische Strahlung aufgrund von Lumineszenzeffekten auszusenden. Nachfolgend werden spezielle Effekte der Lumineszenz beispielhaft dargelegt.

Bei dem Prinzip der Fluoreszenzmessung wird eine Substanz mit monochromatischer, elektromagnetischer Strahlung einer bestimmten Wellenlänge bestrahlt. Verfügt die Substanz über Fluorophore und besitzen die eingestrahlten Photonen die molekülspezifische Energie zur Anregung von Elektronen, so werden die Photonen absorbiert, indem Elektronen von einem Grundzustand in einen höheren energetischen Zustand wechseln. Nach einer für Fluoreszenzeffekte typischen Dauer von bis zu ca. 1µs verlässt das angeregte Elektron seinen höheren energetischen Zustand und kehrt in den Grundzustand zurück. Dabei sendet es die überschüssige Energie in Form von Photonen einer bestimmten Wellenlänge aus. Diese Wellenlänge liegt üblicherweise 20 bis 50 nm oberhalb der Anregungswellenlänge und ist auf Energieverluste durch nicht-strahlende Effekte innerhalb der Energiebänder des Moleküls zurückzuführen. Dieser Unterschied in der Wellenlänge wird üblicherweise als Stokes-Shift bezeichnet, siehe *z.B. "*Topics in Fluorescence Spectroscopy", Vol. 1-11, Joseph R. Lakowicz. Gleichzeitig gibt es aber auch Stoffe, bei denen der Unterschied zwischen Anregungs- und Emissionswellenlänge wesentlich größer ist. Ein Beispiel stellt hier p-cresol dar. Die Anregungswellenlänge dieses Stoffes liegt bei ca. 300 nm, während die Emissionswellenlänge bei ca. 600 nm liegt. Anregungs- und Emissionswellenlänge sind charakteristische Eigenschaften für ein Molekül. Dementsprechend können über die Analyse des Absorptions- und des Emissionsspektrums bei Lumineszenzeffekten wie der Fluoreszenz Rückschlüsse auf die Substanzen innerhalb einer Flüssigkeit gezogen werden.

Die Bestimmung des Gehaltes von Harnstoff bzw. toxischen Substanzen im klein- und mittelmolekularen Bereich sowie im Bereich proteingebundener Substanzen im Dialysierflüssigkeitsabfluss bietet eine umfassende Überwachung des Dialyseverlaufs. Allerdings müssen dazu bis heute Proben manuell dem System entnommen werden und zur chemischen Analyse an ein entsprechend ausgerüstetes Labor weitergereicht werden.

In der EP 2 510 958 wird offenbart, dass Proben aus der Dialysierflüssigkeit genommen und auf optischem Wege untersucht werden um die Dialysebehandlung eines Patienten zu überwachen.

Die EP 2 397 167 A1 offenbart eine Vorrichtung, mit der kontinuierlich während der Therapie eine Aussage über den Gehalt bzw. die Änderung des Gehalts von mittelgrossen Molekülen im Bereich von 500 bis ca. 50000 Da in Abfluss der Dialysierflüssigkeit gemacht werden kann. Ein Rückschluss auf die Reinigungsleistung der Therapie ist in Echtzeit möglich. Zur Bestimmung dieser Inhaltsstoffe von Abfallprodukten in der abfließenden Dialysierflüssigkeit wird die Methode der Lumineszenzmessung und / oder einer Kombination aus Lumineszenz- und Absorbanzmessung verwendet.

Zur kontinuierlichen Überwachung der Hämodialyse kann die Änderung der Leitfähigkeit durch die Hydrolyse von Harnstoff und/oder anderen wichtigen Molekülen in der Dialysierlösung ausgewertet werden. Die Kalibration von Leitfähigkeitssensoren speziell für diese Applikation ist sehr mühselig, da Einflüsse auf die Leitfähigkeit auch aus anderen Quellen entstehen können. Eine nach diesem Prinzip funktionierende Messeinrichtung ist *Biostat*© *Urea Monitor* von Baxter.

Des Weiteren kann die kontinuierliche Überwachung einer Hämodialyse über optische Absorbanzmessung realisiert werden, bei der die Transmission der Dialysierflüssigkeit im Wesentlichen durch Harnsäure und andere kleinmolekulare Stoffe beeinflusst wird. Eine solche Messeinrichtung ist in der EP 1 083 948 B1 beschrieben. Jedoch ist damit keine Aussage über Moleküle mittlerer Größe wie β₂-Mikroglobulin, anderen Peptiden bzw. kleinen Proteinen sowie proteingebundenen Substanzen wie Indoxyl Sulfate, Indole-3-Essigsäure oder p-cresol bzw. p-cresyl Sulfate, welche maßgeblich an dem Krankheitsverlauf der Urämie verantwortlich sind, möglich.

Die Erfindung löst das Problem der genauen Bestimmung von Abfallstoffen während einer Hämodialyse.

Ausführungsbeispiele der Erfindung verbinden die Methode einer Lumineszenzmessung und / oder einer Kombination aus Lumineszenz- und Absorbanzmessung mit einer Dialysemaschine zur Bestimmung der Inhaltsstoffe von Abfallprodukten im Dialysierflüssigkeitsabfluss. Dabei kann die Bestimmung der Konzentration einer bestimmten Substanz oder einer Kombination von Substanzen innerhalb der Dialysierflüssigkeitsabfälle direkt von der Zusammensetzung der aus dem Dialysator während einer Dialysebehandlung herausfließenden Dialysierflüssigkeit abhängen.

Mit der Erfindung ist die Realisierung einer Vorrichtung zur genauen Bestimmung von Abfallstoffen und deren Mengen im Dialysierflüssigkeitsabfluss während einer Hämodialyse möglich. Die Erfindung erlaubt die Kombination aus der Anwendung einer Bestimmungstechnik und der Verwendung einer zuverlässigen medizintechnischen Anlage zur Bestimmung von Proteinen/Peptiden (beispielsweise β₂-Mikroglobulin), proteingebundenen Substanzen wie beispielsweise Indoxyl Sulfate oder auch anderen Substanzen, welche in der Dialysierflüssigkeit nach Durchgang durch den Dialysators enthalten sind. Hierdurch ist anhand der Messergebnisse ein Einfluss auf Dialyseparameter wie Dialysatorauswahl, Blutfluss, Dialysierflüssigkeitsfluss, Therapieart u.v.m. möglich.

Weiter wird ein Verfahren zur Bestimmung der Konzentration beschrieben, welches nicht Gegenstand der Ansprüche ist. Dieses Verfahren zeigt die Bestimmung der Konzentration mindestens einer lumineszenten urämischen Substanz, insbesondere von Indoxyl Sulfate, im Abfluss einer extrakorporalen, einen Dialysator umfassenden Blutwäscheeinheit, mit folgenden Schritten auf: Messen mindestens eines Inhaltsstoffes und optional dessen Konzentration in einer aus dem Dialysator herausströmenden Dialysierflüssigkeit während einer Dialysebehandlung; spektrales Untersuchen der Dialysierflüssigkeit mittels mindestens einer oder mehreren der folgenden Messmethoden: Lumineszenzmethode; Fluoreszenzmethode; Kombination aus Absorbanzmethode und Lumineszenzmethode; Kombination aus Absorbanzmethode und Fluoreszenzmethode; und Ermitteln mindestens eines dialysespezifischen Parameters wie beispielsweise Blutfluss, Dialysierflüssigkeitsfluss, Ultrafiltrationsrate, Therapieart, Therapiedauer usw.

Das nicht beanspruchte Verfahren kann z.B. während einer laufenden Therapie angewendet werden, um in Echtzeit den mindestens einen Inhaltsstoff innerhalb der Dialysierflüssigkeit und / oder dessen Konzentration zu bestimmen.

Hierbei können beispielsweise während einer Therapie in Echtzeit relative Änderungen des mindestens einen Inhaltstoffes oder eines Stoffgemisches im proteingebundenen Bereich, z.B. anhand eines Kt/V_{proteingebundene Substanzen} oder einer prozentualen Entfernungsrate / -menge, bestimmt werden.

Die relativen Änderungen des mindestens einen Inhaltsstoffes oder des Stoffgemisches im proteingebundenen Bereich können z.B. anhand eines Verhältnisses Kt/V_{proteingebundene Substanzen} oder einer prozentualen Entfernungsrate / -menge, bestimmt werden.

Die Ergebnisse der spektralen Untersuchungen können beispielsweise zusammen mit spezifischen Messparametern wie Anregungswellenlänge, Emissionswellenlänge, Intensitätsprofil der Anregungswellenlänge, und/oder Intensitätsprofil der Emissionswellenlänge dargestellt werden, um hieraus auf den Gesamtgehalt eines Stoffes oder eines Stoffgemisches in der aus dem Dialysator austretenden Dialysierflüssigkeit zurückzuschließen.

Ferner können die Ergebnisse der spektralen Untersuchungen z.B. mit Dialyseparametern wie Dialysierflüssigkeitsfluss, Blutfluss, Dialysator etc. dargestellt werden, um auf den Gehalt eines Stoffes oder eines Stoffgemisches auf der Blutseite des Dialysators zurückzuschließen.

Die Messung der Dialysierflüssigkeit kann kontinuierlich, regelmäßig oder sporadisch während einer Dialysetherapie durchgeführt werden.

Hierbei kann vorgesehen sein, eine spektralphotometrische Untersuchung mittels UV-Licht durchzuführen. Die verwendete(n) Anregungswellenlänge(n) für die Fluoreszenzmessung können z.B. im Bereich von 190 bis 750 nm, vorzugsweise im Bereich von 200 bis 300 nm und insbesondere im Bereich von 250 bis 300 nm liegen.

Die Emissionswellenlänge der Fluoreszenzmessung kann im Bereich von 190 bis 750 nm, vorzugsweise im Bereich 300 bis 500 nm liegen.

Die Anregungswellenlänge liegt bei einer Kombination aus einer Fluoreszenz- und einer Absorbanzmessung z.B. im Bereich von 190 bis 750 nm, vorzugsweise im Bereich 200 bis 300 nm und insbesondere im Bereich von 250 bis 300 nm.

Es kann eine Beurteilung und Änderung von mindestens einem patientenindividuellen Behandlungsparameter auf der Basis der Analyse der Messergebnisse aus Fluoreszenzmessung oder der Kombination aus Fluoreszenz- und Absorbanzmessung aus vorhergehenden Therapien, früher gewonnenen Messergebnissen bzw. in Echtzeit vorgenommen werden.

Weiterhin kann mindestens ein Parameter für die Dialysebehandlung in Abhängigkeit von dem Messergebnis angepasst werden.

Das Messergebnis kann in Verbindung mit anderen dialysespezifischen Parametern angepasst werden, wobei die Anpassung automatisch oder manuell vorgenommen werden kann.

Bei einem oder mehreren Ausführungsbeispielen der erfindungsgemäßen Vorrichtung zur Bestimmung der Konzentration mindestens einer lumineszenten urämischen Substanz, insbesondere von Indoxyl Sulfate, im Abfluss einer extrakorporalen, einen Dialysator umfassenden Blutwäscheeinheit, ist eine Messeinrichtung zur Messung mindestens eines Inhaltstoffes und optional dessen Konzentration in einer aus dem Dialysator herausströmenden Dialysierflüssigkeit während einer Dialysebehandlung vorgesehen. Die Messeinrichtung kann eine Lichtquelle zum Bestrahlen der Dialysierflüssigkeit und einen Lumineszenz- oder Fluoreszenzdetektor zum Erfassen von Lumineszenz- oder Fluoreszenzstrahlung, die von der bestrahlten Dialysierflüssigkeit erzeugt wird, enthalten.

Bei einem oder mehreren Ausführungsbeispielen kann die Vorrichtung ein optisches Element aufweisen, das zwischen der Dialysierflüssigkeit und dem Fluoreszenz- oder Lumineszenzdetektor vorgesehen ist. Das optische Element kann z.B. als ein optisches Gitter oder als Filtereinrichtung ausgebildet sein.

Die Lichtquelle kann bei einem oder mehreren Ausführungsbeispielen dazu ausgelegt sein, monochromatisches Licht, optional im Bereich λ = 200 nm bis 500 nm, auszusenden.

Zur Optimierung der Signalqualität ist es außerdem vorteilhaft, die von der Lichtquelle ausgesendeten Lichtstrahlen zu führen, um Streulicht im Messraum zu reduzieren. Streulicht ist ein in der optischen Messtechnik bekanntes Phänomen, welches insbesondere bei Absorption und Fluoreszenzmessungen zu erheblichen Signalstörungen bzw. Signalverfälschungen führen kann. Aus diesem Grund ist der Einbau von Elementen zur Strahlengangsführung wie z.B. Linsen sowohl unmittelbar hinter der Lichtquelle aber vor der Probenanregung (z.B. zwischen den Komponenten 1 und 2 oder zwischen den Komponenten 2 und 3 bzw. auch zwischen den Komponenten 2 und 6) bzw. nach der Probenanregung aber vor dem Fluoreszenzdetektor (z.B. zwischen den Komponenten 3 und 4 bzw. zwischen den Komponenten 4 und 5) zweckmäßig.

Die Vorrichtung kann bei einem oder mehreren Ausführungsbeispielen einen optischen Strahlengangteiler und einen Referenzdetektor enthalten, wobei ein Teil des von der Lichtquelle erzeugten Lichts auf den Referenzdetektor ablenkbar und dort detektierbar ist.

Desweiteren hat sich gezeigt, dass Detektoreinrichtungen zur Messung von Fluoreszenz in dem genannten Bereich hochgradig sensibel auf Temperaturänderungen reagieren. Neben der Änderung der Umgebungstemperatur, welche sich auf die Signalaufnahme des Detektors auswirkt und somit kompensiert werden muss, ist auch die Erhitzung des Fluoreszenzdetektors aufgrund der Stromversorgung und den damit entsprechenden Strömen durch den Detektor selber ein wichtiger Aspekt. Der Fluoreszenzdetektor ist deshalb bei einem, mehreren oder allen Ausführungsbeispielen mit einer entsprechenden Temperaturerfassungseinrichtung und gegebenenfalls mit einer Reglereinrichtung ausgestattet, die in der Lage ist, die Eigenerwärmung, z.B. bedingt durch die Stromversorgung der Detektoreinrichtung, zu erkennen und entweder mittels geeigneter Korrekturverfahren das Messsignal oder Fluoreszenzsignal entsprechend zu korrigieren, oder beispielsweise durch entsprechende Messalgorithmen, z.B. gepulsten Betrieb, nur kurze Messperioden vorsieht, so dass lange Messperioden und somit eine Eigenerwärmung ausgeschlossen oder zumindest reduziert werden können.

Des weiteren hat sich herausgestellt, dass neben der Umgebungstemperatur, welche die Eigenschaften des Fluoreszenzdetektors beeinflussen kann und somit das Fluoreszenzsignal verfälscht, auch die Temperatur der Probenlösung - in diesem Fall z.B. die verbrauchte Dialysierflüssigkeit - die Fluoreszenzeigenschaften der fluoreszierenden Substanzen beeinflusst. Um diese Einflussquelle kontrollieren zu können, ist die Messung der Temperatur in der Messlösung, d.h. hier z.B. der Dialysierflüssigkeit, sinnvoll. Dieses kann zum Einen bei einem, mehreren oder allen Ausführungsbeispielen durch die Integration eines Temperatursensors in die Messküvette und somit den Fluoreszenzsensor geschehen. Zum Anderen ist bei einem, mehreren oder allen Ausführungsbeispielen aber auch die externe Messung der Temperatur durch einen separaten Temperaturmesser vorgesehen, welcher vor oder nach dem Fluoreszenzsensor im Fluss der Messflüssigkeit, d.h. z.B. der Dialysierflüssigkeit, angeordnet ist. Die Temperatur der Messlösung stellt bei diesen Ausführungsbeispielen einen wichtigen Parameter zur optischen Charakterisierung der verbrauchten Dialysierflüssigkeit dar.

Bei einem, mehreren oder allen Ausführungsbeispielen ist deshalb ein Temperatursensor zur Bestimmung der Temperatur in der Dialysierflüssigkeit, d.h. der Probenlösung, und / oder ein Temperatursensor zur Bestimmung der Temperatur der Detektionseinrichtung vorhanden. Bei der Auswertung und Beurteilung der Signale des Fluoreszenzdetektors wird die ermittelte Temperatur der Dialysierflüssigkeit und / oder der Detektionseinrichtung berücksichtigt, um die Signale richtig deuten zu können.

Bei einem oder mehreren Ausführungsbeispielen kann der Lumineszenz- oder Fluoreszenzdetektor winklig wie etwa orthogonal zum ursprünglichen Strahlengang der Lichtquelle angeordnet sein, so dass er nicht direkt im Pfad des Bestrahlungslichts liegt.

Bei einem oder mehreren Ausführungsbeispielen kann ein Absorptionsdetektor vorgesehen sein, so dass zusätzlich die Absorption des durch die Dialyseflüssigkeit hindurchgetretenen Lichts detektierbar ist.

Nachfolgend wird die Erfindung unter Bezugnahme auf die Zeichnungen näher beschrieben.
Fig. 1 zeigt ein Ausführungsbeispiel einer erfindungsgemäßen Vorrichtung,
Fig. 2 stellt den Intensitätsverlauf über die Wellenlänge für die Anregungs- und Emissionswellenlänge von Proteinen/Peptiden oder anderen Substanzen beispielhaft dar,
Fig. 3 zeigt ein Ausführungsbeispiel eines Aufbaus eines bei Ausführungsbeispielen der erfindungsgemäßen Vorrichtung oder des nicht gesondert beanspruchten Verfahrens einsetzbaren Sensors zur Fluoreszenzmessung, und
Fig. 4 zeigt ein weiteres Ausführungsbeispiel eines bei Ausführungsbeispielen der erfindungsgemäßen Vorrichtung oder des nicht beanspruchten Verfahrens einsetzbaren Aufbaus eines Sensors zur Fluoreszenzmessung.

Im Folgenden wird ein Ausführungsbeispiel eines Aufbaus mit einer Kombination aus Fluoreszenz- und Absorbanzmessung erläutert.

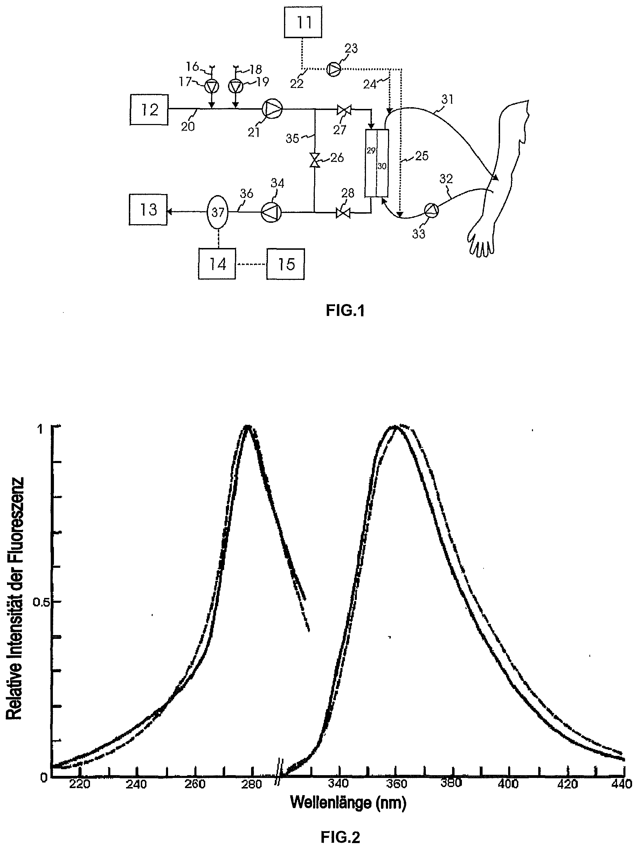

FIG.1 zeigt den Dialysierflüssigkeitskreislauf eines Ausführungsbeispiels einer erfindungsgemäßen Vorrichtung in Form einer Dialysemaschine mit einer zusätzlichen Messeinheit, die im Dialysierflüssigkeitsabfluss angeordnet ist. Das Blut des Patienten wird dabei extrakorporal durch ein Schlauchsystem 32 in die blutseitige Kammer eines Dialysators 30 geführt. Nachdem das Blut den Dialysator 30 passiert hat, wird es über ein Schlauchsystem 31 dem Patienten wieder zugeführt. Der Blutfluss wird dabei durch eine Blutpumpe 33 gesteuert.

Die Dialysierflüssigkeit besteht aus verschiedenen Inhaltsstoffen, welche in Wasser gelöst sind. Daher verfügt die Dialysemaschine über einen Wassereinlass 12, zwei Konzentratzuführungen 16, 18 und zwei Konzentratpumpen 17, 19. Der Wasserfluss bestimmt zusammen mit dem Konzentratfluss die Zusammensetzung der Dialysierflüssigkeit. Über den Dialysierflüssigkeitskreislauf 20 wird die Dialysierflüssigkeit einer von der Blutkammer 30 durch eine semipermeable Membran getrennten Dialysierflüssigkeitskammer 29 des Dialysators zugeführt. Die Dialysierflüssigkeit wird dabei durch eine Dialysierflüssigkeitspumpe 21 dem Dialysator zugeführt und über eine weitere Pumpe 34 zusammen mit dem Ultrafiltrat dem Dialysator entzogen. Eine Bypass-Verbindung ist zwischen den Pumpen 21 und 34 angebracht. Des Weiteren werden Ventile 26, 27 und 28 zur Steuerung des Dialysierflüssigkeitskreislaufes eingesetzt. Über das Schlauchsegment 36 wird die Dialysierflüssigkeit nach dem Durchgang durch den Dialysator einem Fluoreszenzsensor 37 zugeführt, der entweder eine reine Fluoreszenzmessung oder eine Kombination aus Fluoreszenz- und Absorbanzmessung durchführt. Der Fluoreszenzsensor 37 führt die optischen Messungen durch und sendet die Ergebnisse anschließend über ein Interface an eine zugehörige Auswerteeinrichtung 14. Anschließend werden die ausgewerteten Daten z.B. über einen Bildschirm oder Drucker ausgegeben. Nach der Messung wird die Dialysierflüssigkeit einem Abfluss 13 zugeführt.

Die gepunkteten Linien 22, 24 und 25 stellen optionale Komponenten des Systems für die Durchführung einer Hämodialfiltration dar, bei der die Substitutionsflüssigkeit aus einer entsprechenden Quelle 11 ankommt und über das Schlauchsystem 22 mit Hilfe einer Pumpe 23 in den Blutkreislauf des Patienten gefördert wird. Im Falle der Wahl einer Option "post dilution" wird das Substituat dem Blutkreislauf nach Durchlaufen durch den Dialysator-Pfad 24 zugeführt, wohingegen es bei der Wahl einer Option "pre dilution" vor dem Durchlaufen des Dialysator-Pfads 25 zugeführt wird. Im Falle der Option "pre-post dilution" werden beide Pfade (Ports) 24 und 25 bei der Zuführung des Substituates verwendet. Der Computer 14 steuert alle Elemente, die in FIG.1 abgebildet sind, und einige weitere Komponenten, die aus Vereinfachungsgründen weggelassen wurden. Des Weiteren bezieht der Computer 14 Informationen aus der Dialysemaschine, welche mathematisch mit den Ergebnissen der optischen Messeinrichtung verknüpft werden können. Die Position der Sensoreinheit 37 in FIG.1 ist nicht auf die abgebildete Anordnung beschränkt. Wesentlich ist, dass sich die Messeinheit, also z.B. die Sensoreinheit 37, im Dialysierflüssigkeitsabfluss befindet, d.h. an einer beliebigen Position in dialysierflüssigkeitstseitiger Flussrichtung gesehen hinter dem Dialysator 29.

FIG. 2 zeigt einen typischen Verlauf der relativen Intensität der Fluoreszenz in Abhängigkeit von der Anregungs- und Emissionswellenlänge bei einer Fluoreszenzmessung. Der Ausschlag der Intensität bei niedrigen Wellenlängen im Bereich von etwa 270 bis 290 nm entspricht dabei dem Intensitätsprofil der Anregungswellenlänge, während der Anstieg der Intensität bei höheren Wellenlängen von etwa 340 bis 400 nm das Intensitätsprofil der Emissionswellenlänge darstellt. Die Verschiebung der maximalen Intensität von Anregungs- zu Emissionswellenlänge wird *Stokesshift* genannt und ist auf nicht-strahlende Übergänge innerhalb des Moleküls zurückzuführen. Die durchgezogene Linie beschreibt die Spektren für feste Umgebungsbedingen (pH, T, C usw.). Durch die Änderung der Umgebungsbedingungen (pH-Wert der gelösten Flüssigkeit, Temperatur, usw.) können sich beide Spektren ändern. Dies ist durch die gestrichelte Linie angedeutet.

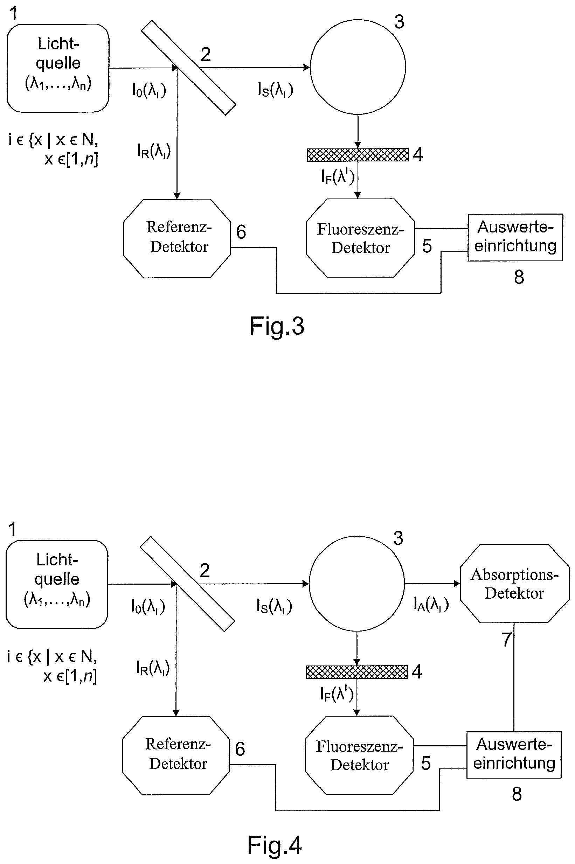

FIG. 3 stellt den Aufbau einer bei Ausführungsbeispielen der erfindungsgemäßen Vorrichtung oder des nicht beanspruchten Verfahrens einsetzbaren Sensoreinheit mit Fluoreszenzmessung dar. Grundsätzlich kann aber auch jede andere Sensoreinheit für eine Lumineszenzmessung derart aufgebaut sein. Von einer Lichtquelle 1 wird elektromagnetische Strahlung l₀(λ) eines bestimmten Intensitäts- und Wellenlängenprofils, beispielsweise monochromatisches Licht im Bereich λ = 200 nm bis 500 nm, ausgesendet. Dieses wird an einem optischen Strahlengangteiler 2 geteilt. Ein Teil des Lichtes wird auf einen Referenzdetektor 6 abgelenkt und dort detektiert. Der andere Teil des Lichtes, der den Strahlengangteiler 2 ungehindert passiert, bestrahlt eine zu untersuchende Probe 3, welche z.B. die verbrauchte Dialysierflüssigkeit ist oder darstellt. Durch Lumineszenz- bzw. Fluoreszenzeffekte emittiertes Licht verlässt die Probe 3 und wird in alle Richtungen abgestrahlt. Ein Lumineszenz- oder Fluoreszenzdetektor wie etwa ein Photodetektor 5, welcher beispielsweise winklig wie etwa orthogonal zum ursprünglichen Strahlengang der Lichtquelle 1 sitzt, detektiert das durch die Probe ausgestrahlte Licht. Mit dem Lumineszenz- oder Fluoreszenzdetektor 5 und dem Referenzdetektor 6 ist eine Auswerteeinrichtung 8 verbunden, die die vorstehend und/oder nachstehend beschriebene Auswertung wie etwa die Analyse ausführt.

Zur verbesserten Darstellung der emittierten Photonen können optische Elemente 4 wie beispielsweise optische Gitter oder andere Filtereinrichtungen zwischen Messprobe 3 und Photodetektor 5 vorgesehen sein, die bei einem oder mehreren Ausführungsbeispielen nur für die interessierende Wellenlänge durchlässig sind. Durch die Analyse der von der Messprobe, d.h. der Probe 3, ausgesandten Wellenlänge können Rückschlüsse auf bestimmte Inhaltsstoffe wie z.B. β₂-Mikroglobulin, Indoxyl Sulfate oder anderen Substanzen und auch deren Konzentration gezogen werden.

FIG. 4 zeigt ein weiteres Ausführungsbeispiel mit einer Kombination aus Lumineszenz- bzw. Fluoreszenz- und Absorbanzmessung zur Bestimmung von Dialysierflüssigkeitsinhaltsstoffen vor dem Abfluss der Dialysierflüssigkeit. Die Abbildung ist ähnlich wie in FIG. 3, wobei die vorstehende Beschreibung von Komponenten auch für das Ausführungsbeispiel gemäß Fig. 4 zutrifft, soweit gleiche Bezugszeichen verwendet sind.

Bei dem Ausführungsbeispiel gemäß FIG. 4 ist die Sensoreinheit mit einem zusätzlichen Absorbanzdetektor (Absorptionsdetektor) 7 gekoppelt, welcher bei einem oder mehreren Ausführungsbeispielen auf der optischen Achse der Lichtquelle 1 sitzt. Der Absorptionsdetektor 7 ist ebenfalls mit der Auswerteeinrichtung 8 verbunden. Die Kombination aus Absorbanzmessung bei gleichzeitiger Lumineszenz- bzw. Fluoreszenzmessung ermöglicht die Messung mittelmolekularer Stoffe wie β₂-Mikroglobulin bei gleichzeitiger Überprüfung der Reinigungsleistung kleinmolekularer Stoffe durch die Absorbanzmessung. Auch hier kann die Wellenlänge λ z.B. im Bereich von 200 bis ca. 500 nm variabel sein und beträgt bei einem oder mehreren Ausführungsbeispielen λ = 280nm. Ansonsten entspricht das Prinzip der Messung den vorstehenden Ausführungen zum Ausführungsbeispiel gemäß Fig. 3.

Ein wichtiger Aspekt betrifft die Lichtführung. Hierfür kann mindestens ein Element zur Strahlengangsführung vorgesehen sein.

Dieses mindestens eine Element zur Strahlengangsführung kann z.B. mindestens eine Linse unmittelbar hinter der Lichtquelle, aber vor der Probenanregung und/oder mindestens eine Linse nach der Probenanregung, aber vor dem Lumineszenz- oder Fluoreszenzdetektor aufweisen.

Zusätzlich zu den vorstehend beschriebenen Ausführungsbeispielen sind weitere Ausführungsbeispiele des nicht beanspruchten Verfahrens und der erfindungsgemäßen Vorrichtung möglich. Beispielsweise kann der Sensor auch als Ein-Strahl-Fotometer ausgeführt werden. Bei einem solchen Ausführungsbeispiel können die Elemente 2 und 6 der Figuren 3 und 4 entfallen, so dass kein Referenzsignal generiert wird. Ebenfalls kann die Lichtquelle 1 in verschiedenen Typen ausgeführt werden. So ist z.B. eine Ausführung als monochromatische oder auch als polychromatische Lichtquelle denkbar.

Ferner kann die optische Filtereinrichtung 4 sowohl als Bandpass mit einem einzigen Passbereich als auch mit mehrfachem Passbereich ausgeführt werden. Durch die Variation der Lichtquelle 1 bzw. der Filtereinrichtung 4 können Moleküle unterschiedlicher Art detektiert werden, was eine Beurteilung der Entfernungsrate toxischer Substanzen aus dem Blut ermöglicht. Diese Variation kann erfolgen als feste Einstellung für einen bestimmten Sensor. Ein Sensor kann aber auch so ausgelegt werden, dass ein Wechsel von Anregungs- bzw. Detektionswellenlängen während einer Therapie möglich ist, z.B. durch Verschieben eines Filterhalters oder Drehen eines Filterrades.

Darüber hinaus ist die Verwendung von mindestens einer, oder vorzugsweise zwei oder mehreren anregungsseitigen Wellenlängen vorteilhaft, die entweder durch eine polychromatische Lichtquelle mit mehreren wellenlängenselektiven Elementen, oder durch mehrere monochromatische Lichtquellen z.B. LEDs erzeugt werden. Hierdurch kann ein differenziertes Bild über die Zusammensetzung der zu untersuchenden wässrigen Flüssigkeit erhalten werden. Die Verwendung verschiedener Anregungswellenlängen kann fest im Sensor eingerichtet sein, z.B. zwei unterschiedliche Lichtpfade für unterschiedliche Wellenlängen, oder während der Therapie automatisch austauschbar gestaltet sein, z.B. durch Verwendung unterschiedlicher Farb- oder Interferenzfilter, wie oben beschrieben.

Entsprechend ist auch die Verwendung von mindestens einer, vorteilhafterweise zwei oder mehreren emissionsseitigen Wellenlängen möglich, welche entweder durch mehrere wellenlängenselektive Elemente voneinander getrennt, oder von mehreren schmalbandigen Detektoren aufgezeichnet werden. Auch hier liefert die Aufnahme von mindestens zwei Wellenlängen ein differenzierteres Bild von der Zusammensetzung der wässrigen Flüssigkeit. Auch hier kann die Wellenlängenselektivität durch das Austauschen wellenlängenselektiver Elemente vor einem Detektionselement erfolgen, bzw. es können spezielle Detektionselemente für jede Wellenlänge vorgesehen sein.

Die vorliegende Erfindung erlaubt die einfache und zuverlässige Bestimmung von Dialysierflüssigkeitsinhaltsstoffen im Dialysierflüssigkeitsabfluss während einer Dialysebehandlung, um dem Patienten einen adäquaten Behandlungserfolg zu gewährleisten, ohne ihn in zu hohem Maße zu belasten ("über-"dialysieren). Durch die Auswertung der Messergebnisse, welche entweder nur die Ergebnisse optischer Fluoreszenzmessungen oder die Ergebnisse aus der Kombination von optischer Fluoreszenz- und Absorbanzmessungen beinhalten, ist es weiterhin möglich, die Behandlung des Patienten an seine speziellen, individuellen Bedürfnisse anzupassen, indem dialysespezifische Parameter wie Art des Dialysators, Art der Therapieform, Höhe des Dialsatflusses usw. im Sinne des Patientenerfolges angepasst werden.

Bei den beschriebenen Ausführungsformen gemäß den Figuren 3 und 4 kann die Messung der Dialysierflüssigkeit z.B. kontinuierlich über die Behandlungszeit durchgeführt werden. Allerdings ist es auch möglich, diese Messungen in diskreten Zeitintervallen durchzuführen. Die Durchführung der Messungen kann vollständig automatisiert werden, jedoch ist auch eine manuelle Aktivierung der Messung denkbar.

Bei dem durchzuführenden Messverfahren kann es sich z.B. entweder um eine reine Fluoreszenzmessung wie in FIG. 2 oder um eine Kombination aus Fluoreszenz- und Absorbanzmessung gemäß FIG. 3 oder FIG. 4 handeln. Der Vorteil einer Kombination liegt unter anderem in der Tatsache begründet, die Reduktionsrate kleinmolekularer Stoffe, welche mit einer Absorbanzmessung recht gut erfasst werden können, parallel zur Reinigungsleistung proteingebundener oder mittelmolekularer Stoffe bestimmen zu können, und diese Reinigungsleistung ins Verhältnis mit der Reinigungsleistung proteingebundener bzw. mittelmolekularer Stoffe, welche durch die Fluoreszenzmessung ermittelt werden kann, setzen zu können. Aus dieser Kombination können Rückschlüsse nicht nur auf die aktuellen Bestandteile der Dialysierflüssigkeit, sondern auch über Filtereigenschaften des Dialysators, Reinigungsverlauf von Molekülen unterschiedlicher Molekülgröße und Toxizität uvm. erhalten werden.

## Patentansprüche

1. Vorrichtung zur Bestimmung der Konzentration mindestens einer lumineszenten urämischen Substanz im Abfluss einer extrakorporalen, einen Dialysator umfassenden Blutwäscheeinheit, mit einer Messeinrichtung zur Messung mindestens eines Inhaltstoffes in einer aus dem Dialysator herausströmenden Dialysierflüssigkeit während einer Dialysebehandlung,
wobei die Messeinrichtung eine Lichtquelle (1) zum Bestrahlen der Dialysierflüssigkeit und einen Fluoreszenzdetektor (5) zum Erfassen von Fluoreszenzstrahlung, die von der bestrahlten Dialysierflüssigkeit (3) erzeugt wird, enthält und
wobei die Vorrichtung eine Temperaturerfassungseinrichtung enthält, die die Eigenerwärmung des Fluoreszenzdetektors (5) bestimmt.

2. Vorrichtung nach Anspruch 1, wobei der Fluoreszenzdetektor (5) ein Photodetektor ist.

3. Vorrichtung nach Anspruch 1, mit einem optischen Element (4), das zwischen der Dialysierflüssigkeit (3) und dem Fluoreszenzdetektor (5) vorgesehen ist und wobei das optische Element (4) als ein optisches Gitter oder als Filtereinrichtung ausgebildet ist.

4. Vorrichtung nach einem der Ansprüche 1 und 3, bei der die Lichtquelle (1) dazu ausgelegt ist, monochromatisches Licht auszusenden.

5. Vorrichtung nach einem der Ansprüche 1 bis 4, mit einem optischen Strahlengangteiler (2) und einem Referenzdetektor (6), wobei ein Teil des von der Lichtquelle (1) erzeugten Lichts auf den Referenzdetektor (6) ablenkbar und dort detektierbar ist.

6. Vorrichtung nach einem der Ansprüche 1 bis 5, bei der der Fluoreszenzdetektor (5) winklig zum ursprünglichen Strahlengang der Lichtquelle (1) angeordnet ist.

7. Vorrichtung nach einem der Ansprüche 1 bis 6, mit einem Temperatursensor, durch den die Temperatur der Dialysierflüssigkeit detektierbar ist.

8. Vorrichtung nach einem der Ansprüche 1 bis 7, mit einem Temperatursensor, durch den die Temperatur der Messeinrichtung detektierbar ist.

9. Vorrichtung nach einem der Ansprüche 1 bis 8, mit mindestens einem Element zur Strahlengangsführung, wobei das mindestens eine Element zur Strahlengangsführung, mindestens eine Linse unmittelbar hinter der Lichtquelle (1), aber vor der Probenanregung und/ oder eine Linse nach der Probenanregung, aber vor dem Fluoreszenzdetektor (5) aufweist.

10. Vorrichtung nach Anspruch 1, wobei der Fluoreszenzdetektor (5) zusätzlich mit einer Reglereinrichtung ausgestattet ist, die dazu angepasst ist, das Messsignal des Fluoreszenzdetektors (5) mittels geeigneter Korrekturverfahren entsprechend zu korrigieren oder
durch entsprechende Messalgorithmen kurze Messperioden vorzusehen, sodass lange Messperioden und somit eine Eigenerwärmung des Fluoreszenzdetektor (5) reduziert werden.

## Claims

1. Device for measuring the concentration of at least one luminescent uremic substance in the discharge of an extracorporeal dialysis unit comprising a dialyzer, with a measuring device for measuring at least one ingredient in a dialysis fluid flowing out of the dialyzer during a dialysis treatment,
wherein the measuring device comprises a light source (1) for irradiating the dialysis fluid and a fluorescence detector (5) for detecting fluorescence radiation generated by the irradiated dialysis fluid (3), and
wherein the device comprises a temperature detection device for determining the self-heating of the fluorescence detector (5).

2. Device according to claim 1, wherein the fluorescence detector (5) is a photo detector.

3. Device according to claim 1, with an optical element (4) arranged between the dialysis fluid (3) and the fluorescence detector (5) and wherein the optical element (4) is configured as an optical grating or as a filter device.

4. Device according to any of claims 1 and 3, wherein the light source (1) is configured to emit monochromatic light.

5. Device according to any of claims 1 to 4, with an optical beam path divider (2) and a reference detector (6), wherein part of the light generated by the light source (1) can be deflected to the reference detector (6) and can be detected there.

6. Device according to any of claims 1 to 5, wherein the fluorescence detector (5) is arranged at an angle to the original beam path of the light source (1).

7. Device according to any of claims 1 to 6, with a temperature sensor by means of which the temperature of the dialysis fluid can be detected.

8. Device according to any of claims 1 to 7, with a temperature sensor by means of which the temperature of the measuring device can be detected.

9. Device according to any of claims 1 to 8, with at least one element for guiding the beam path, wherein the at least one element for guiding the beam path comprises at least one lens immediately after the light source (1) but before the sample excitation and/or one lens after the sample excitation but before of the fluorescence detector (5).

10. Device according to claim 1, wherein the fluorescence detector (5) further comprises a control device configured to correct the measurement signal of the fluorescence detector (5) appropriately by means of suitable correction procedures or
to provide short measuring periods by corresponding measurement algorithms so that long measuring periods and thus a self-heating of the fluorescence detector (5) are reduced.

## Revendications

1. Dispositif servant à déterminer la concentration d'au moins une substance urémique luminescente dans le flux sortant d'une unité de filtration du sang extracorporelle comprenant un dialyseur, avec un système de mesure servant à mesurer au moins un composant dans un liquide de dialyse sortant du dialyseur au cours d'un traitement par dialyse,
dans lequel le système de mesure contient une source de lumière (1) servant à irradier le liquide de dialyse et un détecteur de fluorescence (5) servant à détecter un rayonnement fluorescent, qui est généré par le liquide de dialyse (3) irradié, et
dans lequel le dispositif contient un système de détection de température, qui détermine le réchauffement propre du détecteur de fluorescence (5).

2. Dispositif selon la revendication 1, dans lequel le détecteur de fluorescence (5) est un photodétecteur.

3. Dispositif selon la revendication 1, avec un élément optique (4), qui est prévu entre le liquide de dialyse (3) et le détecteur de fluorescence (5) et dans lequel l'élément optique (4) est réalisé sous la forme d'une grille optique ou sous la forme d'un système filtrant.

4. Dispositif selon l'une quelconque des revendications 1 et 3, dans lequel la source de lumière (1) est configurée pour émettre une lumière monochromatique.

5. Dispositif selon l'une quelconque des revendications 1 à 4, avec un diviseur de chemin des rayons (2) optique et un détecteur de référence (6), dans lequel une partie de la lumière générée par la source de lumière (1) peut être déviée sur le détecteur de référence (6) et y être détectée.

6. Dispositif selon l'une quelconque des revendications 1 à 5, dans lequel le détecteur de fluorescence (5) est disposé selon un angle donné par rapport au chemin des rayons d'origine de la source de lumière (1).

7. Dispositif selon l'une quelconque des revendications 1 à 6, avec un capteur de température, par lequel la température du liquide de dialyse peut être détectée.

8. Dispositif selon l'une quelconque des revendications 1 à 7, avec un capteur de température, par lequel la température du système de mesure peut être détectée.

9. Dispositif selon l'une quelconque des revendications 1 à 8, avec au moins un élément servant à guider le chemin des rayons, dans lequel l'au moins un élément de guidage de chemin des rayons présente au moins une lentille directement derrière la source de lumière (1), toutefois avant l'excitation d'échantillons et/ou une lentille après l'excitation d'échantillons, toutefois avant le détecteur de fluorescence (5).

10. Dispositif selon la revendication 1, dans lequel le détecteur de fluorescence (5) est équipé en supplément d'un système de régulation, qui est adapté pour corriger de manière correspondante le signal de mesure du détecteur de fluorescence (5) au moyen de procédés de correction adaptés ou
pour prévoir, par des algorithmes de mesure correspondants, des périodes de mesure brèves, si bien que de longues périodes de mesure et ainsi un réchauffement propre du détecteur de fluorescence (5) sont réduits.
